(19) Europäisches Patentamt
European Patent Office
Office européen des brevets

(11) Numéro de publication: **0 151 577**
**B1**

(12) # FASCICULE DE BREVET EUROPÉEN

(45) Date de publication du fascicule du brevet: **08.03.89**

(51) Int. Cl.⁴: **C 07 H 15/26,** A 61 K 31/70

(21) Numéro de dépôt: **84901776.9**

(22) Date de dépôt: **27.04.84**

(86) Numéro de dépôt international: **PCT/FR 84/00119**

(87) Numéro de publication internationale: **WO 84/04310 (08.11.84 Gazette 84/26)**

(54) **NOUVEAUX DERIVES GLUCIDIQUES DU 5-HYDROXYTRYPTOPHANE, OBTENTION ET APPLICATION COMME MEDICAMENTS.**

(30) Priorité: **29.04.83 FR 8307209**

(43) Date de publication de la demande: **21.08.85 Bulletin 85/34**

(45) Mention de la délivrance du brevet: **08.03.89 Bulletin 89/10**

(84) Etats contractants désignés: **AT BE CH DE GB LI LU NL SE**

(56) Documents cité:
**FR-A-2 317 937**

**The Merck Index, "Ninth Edition", 1976, no. 4769, Merck & Co., Inc. Rahway, N.J. (US). E.K. Witkop: "Precursor of serotonin. Synthesis from 5-benzyloxyindole by Mannich condensation", pages 645, & J. Am. Chem. Soc. 76, 5579 (1954)**
**Monosaccharide Chemistry, R.J. Ferrier and P.M. Collins (Penguin Books), 1972, p. 72**

(73) Titulaire: **OEUVRES HOSPITALIERES FRANCAISES DE L'ORDRE DE MALTE, 4 avenue Marceau, F-75008 Paris (FR)**

(72) Inventeur: **MESTER DE PARAJD, Laszlo, 3, parc de Bearn, F-92210 Saint Cloud (FR)**
Inventeur: **MESTER DE PARAJD, Madeleine née SZADECZKY- KARDOSS, 3, parc de Bearn, F-92210 Saint Cloud (FR)**

(74) Mandataire: **Phélip, Bruno, c/o Cabinet Harlé & Phélip 21, rue de La Rochefoucauld, F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 151 577 B1

LIBER, STOCKHOLM 1989

**Description**

L'invention concerne de nouveaux dérivés glucidiques du 5-hydroxytryptophane, consistant en des N-1-désoxy-2-céto-glycosyl-5-hydroxytryptophane. Elle a également pour objet un procédé pour l'obtention de ces composés ainsi que l'application de ceux-ci comme médicaments et comme agents actifs dans des compositions pharmaceutiques, en particulier pour le traitement de la lèpre.

Le 5-hydroxytryptophane est un composé connu répondant à la formule:

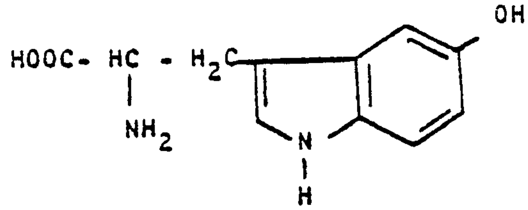

Ce composé existe sous les formes L, D et du mélange D, L. Il peut être préparé en faisant agir une enzyme, la tryptophane-hydroxylase, sur le tryptophane, acide aminé de formule:

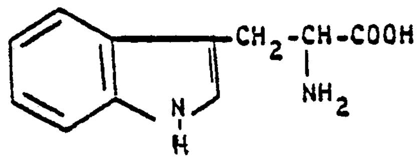

Sa synthèse chimique a été également effectuée: voir A. EK et B. WITKOP dans J. Am. Chem. Soc. 76, 5579 (1954). Le 5-hydroxytryptophane (en abrégé 5-HTP) est déjà proposé comme médicament, en particulier à titre de précurseur de la sérotonine: voir par exemple Dictionnaire VIDAL, Ed. 1980, p. 973, la spécialité disponible sur la marché sous le dénomination PRETONINE, ou MERCK Index, 9ème Edition, p. 645, composé n°4769.

On trouvera encore des indications précises sur la structure du 5-hydroxytryptophane dans l'article de Akio WAKAHARA et al, Bulletin of the Chemical Society of Japan, vol. 46, 2475 - 2480 (1973).

La présente invention concerne de nouveaux dérivés glucidiques du 5-hydroxytryptophane (5-HTP) dans lequel l'un des atomes d'hydrogène du groupe amine primaire du 5-hydroxytryptophane est substitué par un groupe désoxy-1 céto-2 sucre. La substitution s'effectue sur l'atome de carbone 1 du sucre.

Des exemples préférés de sucres convenant pour les composés de l'invention sont les suivants:

D-glucose (hexose),
D-ribose (pentose),
L-fucose (méthylpentose),
melibiose (disaccharide),
erythrose (tetrose),

Lorsque le groupe glucidique est dans sa forme ouverte, les nouveaux dérivés peuvent être en particulier représentés par la formule:

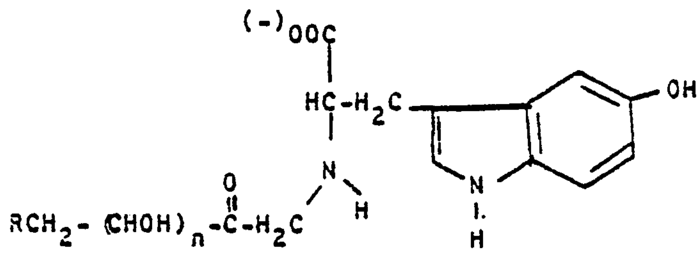

dans laquelle R représente un atome d'hydrogène ou un groupe hydroxyle et n est un nombre entier de 1 à 5.

Un composé particulièrement préféré selon l'invention est le N-1-désoxyfructo-5-hydroxytryptophane qui dérive du D-glucose et du 5-HTP et peut être représenté par la formule:

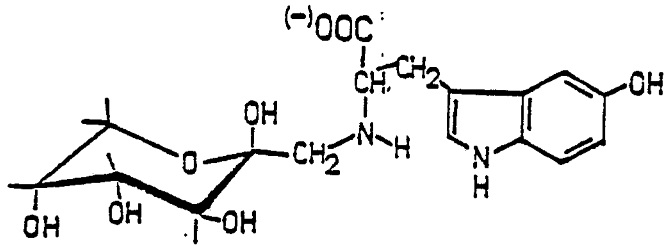

Les composés de l'invention peuvent être préparés par un procédé basé sur une réaction de type Amadori et caractérisé en ce qu'on met en présence au sein d'un solvant le 5-hydroxytryptophane et le sucre

correspondant au dérivé sucré à préparer.

Les conditions générales de la réaction d'Amadori sont bien connues de l'homme de l'art et sont notamment décrites dans "Methods in Carbohydrate Chemistry Vol. 2, Academic Press, N.Y. 1963, page 99", ainsi que dans l'article de John E. Hodge intitulé "The Amadori rearrangement" dans "Advances in Carbohydrate Chemistry, vol. 10, Acad. Press 1965, p. 169". Toutefois, les conditions de mise en oeuvre du procédé de l'invention se distinguent de celles qui sont utilisées habituellement pour ce genre de réaction d'Amadori, lesquelles imposent la présence d'un agent donneur de protons, tel qu'un acide, dans le milieu réactionnel. Selon l'invention, le procédé est grandement simplifié par le fait qu'il est inutile de recourir à un tel agent donneur de protons et qu'il suffit de mettre en présence dans un solvant approprié le 5-HTP et le sucre choisi jusqu'à obtention du dérivé désoxy-1 céto-2 sucre voulu. Le groupe carboxyle du 5-HTP procure en effet les conditions réactionnelles favorables à la formation du dérivé du 5-HTP selon l'invention. Bien entendu, on peut aussi travailler en présence d'un acide ou d'une amine.

Le milieu solvant est de préférence anhydre et comprend en général un alcool, tel qu'un alcool inférieur par exemple le méthanol, l'éthanol, le propanol ou l'isopropanol ou le dioxane, le diméthylformamide ou l'acétonitrile ou des mélanges compatibles de tels solvants, ou tout autre solvant usuel pour la synthèse des dérivés Amadori.

Il convient de mettre en présence au moins des quantités équimolaires de 5-HTP et d'aldose, mais pour augmenter le rendement et rendre la réaction complète, il est avantageux d'utiliser un excès de sucre, par exemple des proportions voisines de 1,5 moles de sucre pour 1 mole de 5-HTP. Le composé recherché précipite hors du milieu réactionnel. L'excès de sucre s'élimine ainsi très aisément.

Pour accélérer et favoriser la réaction, il est recommandé de chauffer le milieu réactionnel, en particulier jusqu'à la température d'ébullition du solvant (reflux). La réaction est poursuivie à une telle température jusqu'à devenir complète.

Le procédé décrit ci-dessus est d'application générale et permet de préparer n'importe quel composé désiré selon l'invention en partant de 5-HTP et du sucre approprié. On a indiqué précédemment des composés préférés de l'invention, mais il va sans dire que celle-ci englobe les dérivés obtenus à partir de l'un quelconque des aldoses suivants: le glycérose, le thréose, l'érythrose, le ribose, l'arabinose, le xylose, le lyxose, le gulose, l'idose, le glucose, le mannose, le galactose, le talose, l'allose, l'altrose, le rhamnose, le fucose, le 6-désoxyglucose ainsi que des disaccharides réducteurs tels que le melibiose, le maltose, le cellobiose et le lactose. Parmi les disaccharides réducteurs, on préfère le mélibiose. Avec les autres disaccharides, tels que le maltose, le cellobiose ou le lactose, la vitesse de la réaction et les rendements sont plus faibles, mais le procédé de l'invention est applicable. On notera qu'on peut mettre en oeuvre dans le procédé de l'invention des sucres de la série D ou de la série L. De même les sucres épimères sont utilisables. L'invention englobe les dérivés obtenus quelle que soit la forme isomère optique des sucres de départ.

On notera aussi que les composés de l'invention peuvent être présents sous leur forme acide ou sous forme de sels. Compte-tenu de la destination des composés de l'invention, il est avantageux que les sels soient pharmaceutiquement acceptables. Les sels alcalins et alcalino-terreux, les sels d'ammonium, les sels avec des amines primaires, secondaires ou tertiaires sont utilisables: lés sels de sodium, de potassium, d'ammonium, de magnésium ét d'amines primaires inférieures, telles que l'éthanolamine, sont préférés.

Les nouveaux composés de l'invention sont caractérisés notamment par leur pouvoir réducteur, qui peut être mesuré par le réactif de Tillman ou dichloro-2,6-phénol-indophénol [voir la référencé H.V. EULEUR et al. Arkiv S. Kemi, 12, 85, (1957)]. Les sucres dont ils dérivent sont sans effet sur un tel réactif. Pour apprécier le pouvoir réducteur, on peut également faire appel à d'autres réactifs connus de l'homme de l'art et capables, par exemple, d'agir sur l'acide ascorbique. Cependant, le moyen de caractérisation le plus commode est le réactif de Tillman.

Les nouveaux composés de l'invention, qu'ils soient sous forme libre ou sous forme de leurs sels, sont des agents actifs comme médicaments. Leur principal intérêt est d'être des agents antilépreux.

On a déjà décrit l'activité antilépreuse de certains dérivés de la sérotonine, voir les articles:

P. Jayaraman, P.R. Mahadevan, M. Mester et L. Mester "Inhibition of the incorporation of ($^3$H)-DOPA in Mycobacterium leprae by desoxyfructo-serotonin", Biochem-Pharm., 29, 2526 (1980) et L. Mestér et S. Balakrishnan "Desoxyfructo-serotonin: first human metabolite with antileprosy activity, Acta Leprologica, n°83, 21 (1981)".

Ainsi, l'activité antilépreuse de la désoxyfructosérotonine in vitro et in vivo a-t-elle été déjà décrite. Les dérivés désoxy-1 céto-2 sucre de la sérotonine possèdent d'autres propriétés pharmacologiques: par exemple, ils sont efficaces comme radioprotecteurs et ils inhibent l'agrégation plaquettaire induite par la sérotonine. De tels dérivés, en particulier la désoxyfructo-sérotonine, ont fait l'objet de la demande de brevet français 75 22 577 (publication n° 2 317 937) au nom de l'Etablissement public dit: Agence Nationale de Valorisation de la Recherche (Anvar) ayant pour titre "Nouveaux dérivés de la sérotonine". Elle décrit aussi l'obtention de tels dérivés de la sérotonine par un procédé mettant en jeu la réaction d'Amadori en présence d'un agent donneur de protons.

Les nouveaux composés dé l'invention sont des agents anti-lèpreux plus avantageux que les dérivés de la sérotonine, car, à activité égale, ils ont une action de plus longue durée. Leur toxicité est nettement plus faible dans les mêmes conditions. On donne également ci-après les résultats d'une étude comparative concernant les métabolites des dérivés désoxy-céto-sucres de la sérotonine d'une part et du 5-hydroxytryptophane d'autre part. Ces résultats montrent que les métabolites mis en évidence dans le sang des animaux traités sont

différents, ce qui indique un mécanisme d'action différent. Il apparaît donc que, dans l'action antilèpreuse, les dérivés selon l'invention du 5-hydroxytryptophane ne se comportent pas comme des précurseurs des dérivés correspondants de la sérotonine, contrairement à ce qui aurait pu être prévu étant donné que le 5-hydroxytryptophane seul ést un précurseur de la sérotonine. Lés dérivés désoxy-1 céto-2 sucres du 5-hydroxytryptophane ne sont pas, en revanche, de simples précurseurs des dérivés correspondants de la sérotonine. Leur comportement propre dans l'activité antilèpreuse constitue une caractéristique importante et inattendue de la présente invention.

Ainsi qu'on l'a mentionné précédemment, les nouveaux composés de l'invention sont utiles comme agents actifs de médicaments, surtout comme agents antilèpreux. Ils peuvent cependant recevoir aussi d'autres applications dans des domaines où le 5-hydroxytryptophane est déjà connu comme possèdant une action thérapeutique, par exemple comme psychotropes.

L'invention concerne aussi des compositions pharmaceutiques, contenant une quantité efficace d'au moins un composé de l'invention ou d'un de ses sels physiologiquement acceptables en association avec un véhicule convenant pour l'administration considérée.

Des compositions convenant à l'administration orale se présentent par exemple sous forme de gélules ou de comprimés. Les excipients à utiliser sont traditionnels et entièrement à la portée de l'homme de l'art. D'autres compositions utilisables sont celles convenant à l'administration locale, et qui se présentent par exemple sous forme de lotions ou de pommades. Les lotions à base aqueuse sont faciles à préparer car les nouveaux composés sont aisément solubles dans l'eau. On peut encore utiliser les produits de l'invention en injections sous-cutanées, par exemple dans la technique connue sous le nom de mésothérapie. Une forme galénique intéressante consiste en une composition injectable par voie intraveineuse, qu'on obtient, par exemple, par dissolution du composé actif dans l'eau distillée avec addition éventuelle de tempons ainsi qu'il est bien connu de l'homme du métier.

Pour le traitement de la lèpre, la posologie à employer dépend de la voie d'administration et de la gravité des zones atteintes par la maladie. En règle générale des doses de 20 mg par kg de poids et par jour sont des doses maximales. Les quantités efficaces se situent le plus souvent entre 2 et 10 mg/kg et par jour en administration orale. De telles doses sont à absorber quotidiennement en plusieurs prises.

Des études in vitro et in vivo chez la souris ont mis en évidence l'activité antilèpreuse des nouveaux composés de l'invention. La durée du traitement, avec la posologie mentionnée ci-dessus, pourra évidemment varier dans de larges limites. Des durées prolongées sont rendues possibles grâce à la faible toxicité des nouveaux composés. De plus ceux-ci exercent une action plus durable que les dérivés correspondants de la sérotonaine.

L'invention est encore illustrée, sans être aucunement limitée, par la description ci-après qui concerne des exemples de préparation de composés représentatifs de la présente invention et des résultats d'études pharmacologiques.

**Exemple 1**

Préparation de N-1-désoxyfructo-5-hydroxytryptophane

220 g (1 mole) de 5-hydroxytryptophane et 270 g (1,5 mole) de D-glucose sont chauffés à énviron 64°C dans 10 l de méthanol pendant 8 heures.

On évapore la solution ainsi obtenue à la moitié de son volume. Par addition de 40 l d'acétate d'éthyle on précipite 400 g de désoxyfructo-5-hydroxytryptophane. Si nécessaire, on peut renouveller la précipitacion afin de purifier le produit.

| | | C % | H % | N % |
|---|---|---|---|---|
| Desoxyfructo-5HTP | | | | |
| $C_{17}H_{22}O_8N_2 = 382,36$ | Calculé: | 53,40 | 5,80 | 7,33 |
| | Trouvé: | 53,68 | 6,28 | 7,76 |
| | | 53,78 | 6,08 | 7,45 |

Le produit se caractérise par son pouvoir réducteur mesuré sur le réactif (solution) de Tillman: on trouve que 1/20 mMole réduit 12 ml de solution de Tillman.

Le 1-désoxyfructo-5-hydroxytryptophane est facilement soluble dans l'eau. Il ést insoluble dans le benzène, l'acétaté d'éthyle et le chloroforme.

Les valeurs caractéristiques des spectres RMN du carbone-13 sont indiquées dans le Tableau 1 qui suit.

Il a été également mis en évidence par une variante de la méthode RMN du carbone 13, dite NORD [Voir "Carbon-13 NMR Spectroscopy" J.B. Stothers, Academic Press, 1973] que le carbone en position 2 est quaternaire, ce qui prouve la présence d'un produit Amadori.

**Exemples 2 à 5**

On a préparé d'autres composés de l'invention en chauffant à 64°C énviron, une mole de 5-hydroxytryptophane et 1,5 mole environ de sucre dans le méthanol pour obtenir des 1-désoxy-2-cétoglycosyltryptophanes conformément à la technique de l'exemple 1.

Après migration sur couche mince de cellulose dans un mélange de butanol: eau: acide acétique (5 : 3 : 2), et révélation par la ninhydrine ou par TTC, chacun de ces dérivés glucidiques montre une tache différente du produit de départ.

Les sucres ci-après ont été utilisés comme matières premières: D-ribose (exemple 2), L-fucose (exemple 3), mélibiose (exemple 4), érythrose (exemple 5).

Avec le réactif de Tillman, les volumes de solution de réactif capables d'être réduits par 1 mMole/ 20 sont les suivants:

5-HTP + D-ribose (exemple 2): 12 ml
5-HTP + L-fucose (exemple 3): 10 ml
5-HTP + melibiose (exemple 4): 8 ml
5-HTP + erythrose (exemplé 5): 6 ml

Les amalyses élémentaires ont donné les résultats ci-après:

| | | C % | H % | N % |
|---|---|---|---|---|
| Desoxyribulo-5HTP (Ex. 2) | | | | |
| $C_{16}H_{20}O_7N_2$ = 352,34 | Calculé: | 54,54 | 5,72 | 7,95 |
| | Trouvé: | 54,40 | 6,02 | 7,12 |
| | | 53,35 | 6,02 | 7,08 |
| Desoxyfucosyl-5HTP (Ex. 3) | | | | |
| $C_{17}H_{22}O_7N_2$ = 366,36 | Calculé: | 55,73 | 6,05 | 7,65 |
| | Trouvé: | 55,18 | 6,69 | 6,59 |
| | | 55,92 | 6,57 | 6,32 |
| Desoxymelibio-5HTP (Ex. 4) | | | | |
| $C_{23}H_{32}O_{13}N_2$ = 544,50 | Calculé: | 50,70 | 5,92 | 5,17 |
| | Trouvé: | 48,52 | 6,78 | 4,97 |
| | | 48,80 | 6,64 | 4,97 |
| Desoxyerythro-5HTP (Ex. 5) sous forme de méthanolate | | | | |
| $C_{16}H_{22}O_7N_2$ = 354,35 | Calculé: | 54,23 | 6,26 | 7,91 |
| | Trouvé: | 53,70 | 6,06 | 7,75 |
| | | 53,43 | 5,96 | 7,74 |

Les valeurs caractéristiques des spectres RMN du carbone-13 des divers composés sont rassemblées dans le tableau 1.

**Tableau 1**

**Valeurs caractéristiques des spectres RMN du carbone-13 des dérivés desoxy-1 ceto-2 glycosyl du 5-hydroxytryptophane**

| Composés dans $D_2O$ | 5-HTP | DP-5HTP (Ex. 1) D-Glucose | DRi-5HTP (Ex. 2) D-Ribose | DFu-5HTP (Ex. 3) L-Fucose | DM-5HTP (Ex. 4) Melibiose | DEr-5HTP (Ex. 5) Erythrose |
|---|---|---|---|---|---|---|
| $C_2$ | 126,9 | 127,0 | 126,7 | 127,0 | 127,1 | 127,0 |
| $C_3$ | 106,7 | 107,5 | 107,1 | 107,2 | 107,5 | 107,9 |
| $C_4$ | 113,6 | 113,7 | 113,4 | 113,6 | 113,9 | 113,7 |
| $C_5$ | 149,7 | 149,9 | 149,6 | 149,8 | 150,1 | 149,7 |
| $C_6$ | 112,7 | 112,5 | 112,5 | 112,5 | 112,6 | 112,5 |
| $C_7$ | 103,2 | 103,8 | 103,4 | 103,4 | 103,7 | 103,4 |
| $C_8$ | 132,4 | 132,6 | 132,3 | 132,5 | 132,7 | 132,5 |
| $C_9$ | 128,1 | 128,4 | 127,9 | 128,0 | 128,5 | 128,0 |
| α | 54,4 | 62,7 | 63,6 | 62,6 | 62,3 | 62,4 |
| β | 26,7 | 27,4 | 26,3 | 27,5 | 26,0 | 27,5 |
| $C_1$ | | 50,0 | 50,8 | 50,7 | 50,6 | 50,6 |
| $C_2$ | | 96,2 | 103,7 | 103,6 | 102,5 | 206,3 |
| $C_3$ | | 71,4 | 72,4 | 73,4 | Les autres | 72,8 |

| | | | | | | |
|---|---|---|---|---|---|---|
| $C_4$ | | 70,8 | 73,5 | 76,8 | signaux de | 62,7 |
| $C_5$ | | 69,9 | 78,3 | 79,9 | 60 à 100 ppm | |
| $C_6$ | | 64,0 | | 19,2 | correspondent au melibiose | |
| COOH | 173,2 | 174,2 | 173,7 | 175,6 | 174,2 | 175,6 |

**Etudes pharmacologiques des dérivés glucidiques du 5-hyxdroxytryptophane**

Les études pharmacologiques effectuées avec les dérivés glucidiques du 5-hydroxytryptophane n'ont montré aucune toxicité ou effet secondaire indésirable. La valeur $DL_{50}$ i.p. pour tous ces dérivés a été trouvée voisine de 800 mg/kg et la valeur DL 50 i.v. est supérieure à 5 g/kg.

Les études suivantes ont été exécutées:

1. Profil pharmacologique général du désoxyfructo-5-hydroxy-tryptophane ou DF-5-HTP (composé de l'exemple 2).
2. Profil pharmacologique général du désoxyribulo-5-hydroxytryptophane (composé de l'exemple 3).
3. Activité anti-stress du OF-5 HTP.
4. Activité cardiovasculaire du DF-5 HTP.
5. Activité du DF-5HTP sur l'incorporation de la DOPA dans les Mycobactéries de la lèpre.
6. Activité du DF-5 HTP sur les M. Leprae sur les pattes des souris.
7. Inhibition exercée par le DF-5HTP sur l'enzyme DOPA-oxydase par comparaison avec la désoxyfructosérotonine (DFS).
8. Métabolisme in vivo du DF-5HTP comparé à celui de la DFS.

Les résultats relatifs au profil pharmacologique général du DF-5HTP sont rassemblés dans le tableau 2, qui contient aussi les informations correspondantes relevées dans les mêmes conditions pour le 5-HTP. Les mesures ont été faites à 15, 30 et 60 minutes après l'administration par voie intrapéritonéale (i.p.) à des souris.

Le tableau 3 est un tableau similaire au tableau 2, qui rassemble les résultats concernant le 5-HTP et le désoxyribulo-5HTP (composé de l'exemple 2); les mesures ont été faites 15 minutes après l'administration.

Aux tableaux 2 et 3, les mesures des divers paramètres étudiés ont été faites conformément aux protocoles indiqués dans les références bibliographiques suivantes:

Activité motrice spontanée: MODIGH, K., Central and peripheral effects of 5-hydroxytryptophan on motor activity in mice. Psychopharmacologia (Berlin), 23, 48, 1972. Cadence respiratoire: GARATTINI, S., and VALZELLI, L. Serotonin and cardiovascular system, Chapter 9, in "Serotonin", Elsevier Publ. Co. Amsterdam 1965.

COHEN, Y. and BLANCHARD, P. Influence de la chélation du calcium sur les effets tensionnels de la sérotonine chez le chien. J. Physiol., 52, 569, 1960.

BRAUNWALD, E. Control of myocardial oxygen consumption: Physiologic and clinical considerations. Am. J. Cardiol., 27 (4), 416, 1971.

"Rotarod": KINNARD, Wm. J. jr. and CARR, C.J. A preliminary procedure for evaluation of central-nervous-system depressants. J. Pharmacol. Exptl. Therap., 121, 354, 1957.

Activité anticonvulsivante (MES): % de protection Temps de sommeil au pentobarbital: WYATT, R.J., ZARKONE, V. ENGELMANN, K., DEMENT, W.C., SMYDER, F. and SJOERDSMA, A., Effects of 5-Hydroxytryptophan on the sleep of normal human subjects. Electroencephaly and Clinical Neurophysiology 30, 505 1971.

Catatonie: BOISSIER, J.R. and SIMON, P., Catalepsie... Thérapie, 18, 1257, 1963.

Le tableau 4 contient des indications résumées sur l'activité anti-stress du DF-5HTP comparée à celle du 5-HTP. Les essais ont été réalisés conformément aux protocoles indiqués dans les références bibliographiques ci-après:

BONFILS, S., LIEFOOGHE, GK, ROSSI, G. and LAMBLING, A. L'ulcère de contrainte du Rat blanc. Modifications de la fréquence lésionnelle par différents procédés opératoires et pharmacodynamiques. C.R. Soc. Biol., 151, 1149, 1957.

BONFILS, S., ROSSI, G., LIEFOOGHE, G. and LAMBLING, A., Ulcère expérimental de contrainte, I. Méthodes, fréquence des lésions, modifications par certains procédés techniques et pharmacologiques. Rev. franç. Etudes clin. biol., 4, 146, 1959.

La leucocytose a été induite par injection sous-cutanée de 0,1 ml de lait avant le traitement d'essai, selon le protocole de BREKHMAN, I.I. and DARDYMOV, I.V. Substances of plant origin which increase non specific resistance. Annu. Rev. Pharmacol. 9, 419, 1969.

Au tableau 4, la lettre N désigne le nombre d'animaux (souris). Un astérisque indique une activité importante. Deux astérisques indiquent une possibilité d'activité positive. Dans l'essai d'endurance à la nage, on a observé les souris jusqu'à 540 minutes, la durée moyenne de nage pour les souris témoins étant de 385 $\pm$ 31 minutes.

Le tableau 5, contient des résultats d'essais concernant l'activité cardiovasculaire du DF-5HTP comparée à celle du 5-HTP. Le protocole expérimental était celui décrit dans "Sugar Derivatives of Indolamines. part V: Cardiovascular Effects and "Anti-stress" Activity of Desoxyfructose Derivative of Serotonin and 5-Metho-xy-tryptamin", L. Mester, M. Mester, K.P. Bhargava, C. Labrid, G. Dureng and M. Cheucle, THROMBOSIS RESEARCH, 15, 245, 1979.

Le tableau 6 rassemble des résultats d'essai concernant l'activité du DF-5HTP sur l'incorporation de la DOPA dans les Mycobactéries de la lèpre. Le protocole expérimental était celui décrit dans JAYARAMAN, P., MAHADEVAN, P.R., MESTER, M. and MESTER, L. Inhibition of the incorporation of $^{3}H$-DOPA in Mycobacterium leprae by Desoxyfructo-serotonin. Biochem. Pharm., 29, 2526, 1980.

On a inoculé 5 x $10^{6}$ M. leprae par tube; le milieu était un tampon phosphate à pH 6,8. La quantité de 3H-DOPA était de 1 μ Ci/tube.

Le tableau 7 contient les résultats d'essai concernant l'activité du DF-5HTP sur les M. leprae sur les pattes de souris. Le protocole expérimental était le suivant:

MESTER, L., and BALAKRISHNAN, S., Desoxyfructo-serotonin: First Human Metabolite with Antileprosy Activity. Acta Lepr. N° 83, 21, 1981.

La dose de DF-5HTP était de 0,5 mg par 5 g de nourriture et par souris. Cette dose était administrée tous les jours à partir de la date d'inoculation. Les essais ont porté sur un lot de 24 souris.

D'autres essais ont été entrepris pour mesurer l'inhibition exercée par la DF-5HTP dans le système DOPA-oxydase + DOPA, par comparaison avec la désoxyfructosérotonine (DFS). Pour ce qui concerne l'inhibition de la DOPA-oxydase, on pourra se reporter à la méthode de N.H. Horowitz, M. Fling et et C. Horn "Tyrosinase", dans Methods in Enzymology Vol. XVII - A, p. 615, Editors: Tabor and Tabor, Ac. Press, 1970.

L'effet de la désoxyfructo-sérotonine (DFS) sur les Mycobactéries de la lèpre est attribué à son effet inhibiteur sur la DOPA-oxydase de M. leprae. Une DOPA-oxydase semblable, isolée de champignons, est commercialisée (SIGMA) et a servi comme modèle afin de comparer l'effet inhibiteur de la DFS et du désoxyfructo-5-hydroxytryptophane sur cette DOPA-oxydase. La figure 1 montre que le dérivé sucré du 5-hydroxytryptophane possède un effet inhibiteur propre et différent de celui de la DFS.

**TABLEAU 2**

| | | 5 HTP | | DF-5HTP | |
|---|---|---|---|---|---|
| | | 50 mg/kg (ip) | 100 mg/kg (ip) | 100 mg/kg (ip) | 174 mg/kg (ip) |
| Activité motrice spontanée | | sans effet | d'abord légère augmentation et ensuite diminution légère | légère augmentation | augmentation |
| Cadence respiratoire | | sans effet | augmentation | sans effet | sans effet |
| Hypersensibilité au bruit | | + | ++ | " | " |
| Défécation | | + | ++ | " | " |
| Essai à la tige Rotarod | | Défaut de coordination musculaire observé (Nbre de souris) | | | |
| | Min. | | | | |
| | 15 | 1/5 | 4/5 | 0/5 | 0/5 |
| | 30 | 1/5 | 3/5 | 0/5 | 0/5 |
| | 60 | 0/5 | 3/5 | 0/5 | 0/5 |
| Tamis incliné | | | | | |
| | 15 | | | | |
| | 30 | Pas d'effets | | | |
| | 60 | neurotoxique | " | " | " |
| Activité anticonvulsivante | | | | | |
| MES* | 15 | 20 | 50 | 0 | 20 |
| % protection | 30 | 25 | 33,3 | 0 | 20 |
| | 60 | 25 | - | 0 | 0 |
| Temps de sommeil au pentobarbital % du témoin | | | | | |
| (témoin = 100) | 15 | 285 | 295 | 140 | 93 |
| | 30 | 222 | 276 | 114 | 95 |
| | 60 | 136 | 279 | 103 | 91 |

*MES = acide 2-(N-morpholino) éthane sulfonique

**TABLEAU 3**

| | 5HTP 100 mg/kg i.p. | Désoxy ribulo-5HTP 100 mg/kg i.p. |
|---|---|---|
| Activité motrice spontanée | Légère augmentation | sans effet |
| Cadence respiratoire | Légère augmentation | sans effet |
| Hypersensibilité au bruit | oui | oui (à un degré moindre) |
| Défécation | oui | oui (à un degré moindre) |
| Essai à la tige Rotarod | Défaut de coordination neuromusculaire observé (4 souris sur 5) | Défaut de coordination neuromusculaire présent à un très faible degré (1 souris sur 5) |
| Tamis incliné | Pas d'effet neurotoxique | Pas d'effet neurotoxique |
| Essai de grimpé sur un baton vertical | Sans effet | Sans effet |
| Activité motrice (Photactametre) | Utilisé comme témoin pour comparer le dérivé sucre | Augmentation pendant les 40 premières min. et ensuite diminution |
| Temps de sommeil au pentobarbital % du témoin (Témoin à 100) | 467,7 min. | 309,2 min. |
| Activité anticonvulsivante | | |
| 1. MES | 60 % | 20 % |
| 2.Pentylènetetrazol | 0 % | 0 % |
| Essai à la morphine | Négatif (pas d'antagonisme) | Négatif (pas d'antagonisme) |
| $DL_{50}$ (ip)en toxicité aigue | > 800 mg/kg | > 800 mg/kg |
| Catatonie | non | non |

**TABLEAU 4**
**Activite anti stress de 5-HTP et DF 5-HTP**

| Composés | Dose mg/kg i.p. | Endurance à la nage | Effet anti-ulcère chez le rat (18 heures sous contrainte | | ACTIVITE (Effet sur la leucocytose induite par le lait | Performance à l'essai Rotarod |
|---|---|---|---|---|---|---|
| | | % d'augmentation | % Ulcération | Indice d'Ulcération (N) | Moyenne T.L.C./Cmm (N) | Moyenne (Sec) (N) |
| 1. Témoin | Solution saline | | 90 | 34 (10) | 23866 ± 214 (10) (lait seulement) | 11,3 ± 3,1 (30) |
| 2. 5-HTP | 100 | 40** | 100 | 30 (4) | 10100 ± 68* (10) | 66,2 ± 6,4* (30) |
| 3. DF-5HTP | 174 | 40** | 50 | 10**(4) | 10468 ± 130* (8) | 16,4 ± 2,4 (30) |

(N) - représente le nombre d'animaux
* - activité importante
** - activité positive possible
Pour l'endurance à la nage, les souris ont été observées jusqu'à 540 min, la durée moyenne de nage chez les souris témoins était de 385 + 31 min.
*** - La leucocytose a été induite par 0,1 ml de lait (sous-cutanée) avant traitement avec le médicament.

**TABLEAU 5**
**Effets cardiovasculaires du 5-hydroxytryptophane et de ses dérivés glucidiques**

| Composé | Tension sanguine (en mm Hg) | | | | | Rythme cardiaque (pulsations/min) | | | | | REPONSE CO++ (mm) | | | | |
|---|---|---|---|---|---|---|---|---|---|---|---|---|---|---|---|
| | EFFET Valeur de base | Modification | Mesure à (min) | Pic (min) | Durée (min) | EFFET Valeur de base | Modification | Mesure à (min) | Pic (min) | Durée (min) | EFFET Valeur de base | Modification | Mesure à (min) | Pic (min) | Durée (min) |
| DF 5-HTP* | 76 | - 12 | 1 | 5 | 50 | 180 | - 20 | 30 | 75 | non manifestée à 150 | 28 | + 18 | 30 | 30 | 120 |
| -do-+ | 76 | - 26 | immédiat | - 1 | 30 | 170 | - 20 | 5 | 10 | 45 | 28 | - 8 | 1 | 1 | 45 |
| 5-HTP* | 78 | - 32 | 1 | 15 | 240 | 160 | - 40 | 5 | 30 | non manifestae jusqu'à 240 | 32 | - 24 | 1 | 10 | 105 |
| -do-+ | 76 | - 28 | 1 | 10 | 60 | 140 | + 20 | 1 | 30 | 60 | 32 | + 20 | 5 | 15 | 45 |

*: administration d'une dose de 10 mg par voie cérébroventriculaire
+: injection d'une dose de 100 mg par voie intraveineuse
++: réponse CO = réponse à l'obstruction de la carotide

**TABLEAU 6**

**Inhibition par la desoxyfructo-serotonine (DF-5HT) et la desoxyfructo-5-hydroxy tryptophane (DF-5HTP) de l'incorporation de 3H-DOPA par M. Leprae in vito**

| ESSAIS: | Armadillo M.Leprae N°2275/5 | Armadillo M.Leprae N°2457/103 | Armadillo M.Leprae N°2275/5 | Armadillo M.Leprae N°2457/103 |
|---|---|---|---|---|
| | | | Tués par la chaleur | |
| Tubes frais (témoins) | 5977 | 4215 | 915 | 2855 |
| + DF-5HT: | | | | |
| 1 µg/ml | 2431 | 2575 | 410 | 2110 |
| 5 µg/ml | 2264 | 1485 | 566 | 1640 |
| + DF-5HTP: | | | | |
| 1 µg/ml | 2922 | 2555 | 1008 | 2660 |
| 5 µg/ml | 1599 | 1930 | 732 | 2675 |

Protocole: Chaque tube de 0,5 ml est inoculé de 5 x $10^6$ M. leprae et de 1 µ Ci de $^3$H-DOPA. Après 6 heures, l'incorporation de la DOPA radioactive est enregistrée en DPM selon la technique utilisée dans JAYARAMAN, P., MAHADEVAN, P.R., MESTER, M. and MESTER, L. Inhibition of the Incorporation of $^3$H-DOPA in Mycobacterium leprae by Desoxyfructo-serotonin. Biochem. Pharm., 29, 2526, 1980.

**TABLEAU 7**

**Inhibition de la multiplication de M. Leprae sur les pattes des souris par administration par voie orale de 20 mg/kg de DF-5HTP**

| Mois après inoculation | Témoins | Traité par DF-5HTP |
|---|---|---|
| 4 | 33,8 X | 2,8 X |
| 6 | 38,4 X | Négatif |

Protocole: Sur la patte de chaque souris on a inoculé 5 x $10^6$ M.leprae montrant un index morphologique (I. M.) = 2 %. Les chiffres suivis par X indiquent la multiplication de M. leprae initialement inoculé. (Pour la méthode utilisée voir MESTER, L., and BALAKRISHNAN, S., Desoxyfructo-serotonin: First Human Metabolite with Antileprosy Activity, Acta Lepr. N° 83, 21, 1981.

**TABLEAU 8**

Indication par électrophorèse à haute tension des métabolites de la désoxyfructo-sérotonine et du désoxyfructo-5-hydroxytryptophane radioactifs dans le plasma des hamsters après administration par voie buccale de ces produits

| Produits administrés 2 h plus tôt | Métabolites radioactifs (DPM/ml plasma) | | | |
|---|---|---|---|---|
| | DF-5HT | DF-5HTP + 5HTP | 5-HT | HIAA* + IAA** |
| DF-5HT | 35,000 | - | 5.000 | 5.000 |
| DF-5HTP | 6.000 | 25.000 | 5.000 | 19.000 |
| Produits administrés 5 h plus tôt | | | | |
| DF-5HT | 7.000 | | 2.000 | 2.000 |
| DF-5HTP | 5.000 | 16.000 | 4.000 | 13.000 |
| Produits administrés 24 h plus tôt | | | | |
| DF-5HT | 1.000 | | 500 | 1.000 |
| DF-5HTP | 3.000 | 6.000 | 3.000 | 3.000 |

HIAA* = acide 5-hydroxy indol-acétique
IAA** = acide indol-acétique

La figure 1 est un graphique dans lequel on a porté en abscisses la concentration de l'agent de traitement utilisé et en ordonnées les valeurs de la coloration du milieu mesurée à 540 mμ, celles-ci étant en effet représentatives de la concentration en substance capable de colorer le milieu. En présence d'un inhibiteur, la formation de la coloration est empêchée. Ainsi, plus les valeurs portées en ordonnées sont faibles, meilleure est l'inhibition procurée par le composé essayé. On notera qu'aux concentrations habituellement utilisées, qui sont inférieures à $10^{-4}$M, l'activité inhibitrice du DF-5HTP est comparable à celle de la DFS.

Les essais ci-dessus démontrent in vitro et in vivo l'efficacité du DF-5HTP comme agent antilèpreux. En particulier, l'administration de DF-5HTP empêche la multiplication des mycobactéries M. Leprae sur les pattes de souris.

On a également fait des essais pour apprécier le métabolisme in vivo du DF-5HTP par comparaison avec la DFS. Le tableau 8 contient les résultats obtenus avec la DFS et le DF-5HTP radioactifs et les conditions dans lesquelles l'expérimentation a été réalisée.

La mesure de la radioactivité des diverses fractions isolées du sang des hamsters traités permet de caractériser les métabolites, lesquels sont ainsi isolés et identifiés par électrophorèse. Les métabolites acides HIAA et IAA sont séparés ensemble. L'examen des résultats montre des différences importantes de comportement entre le DF-5HTP et la DFS. Les métabolites acides issus du DF-5HTP sont en beaucoup plus grande quantité que ceux provenant de la DFS. Le DF-5HTP ne se transforme que faiblement en DFS, et contrairement à la DFS, il subsiste une quantité considérable de DF-5HTP non transformé. Ainsi le DF-5HTP ne se comporte pratiquement pas en précurseur de la DFS.

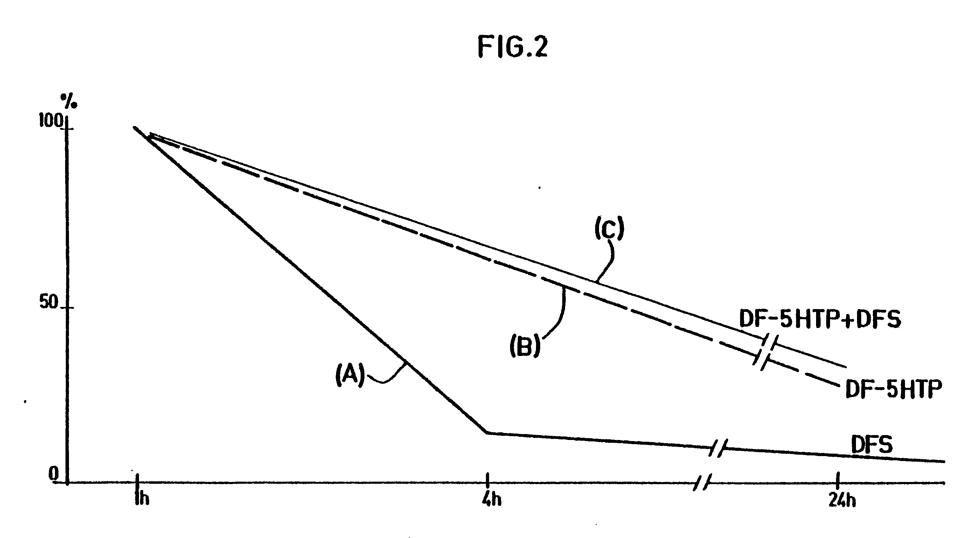

La figure 2 est un graphique illustrant le métabolisme de la DFS et du DF-5HTP dans le plasma du hamster (essai in vivo). On a porté le temps en abscisses (exprimé en heures) et en ordonnées le pourcentage du composé caractérisé dans le plasma. La courbe (A) indique le pourcentage de DFS non transformée, la courbe (B) (en pointillés) représente le pourcentage du DF-5HTP non transformé et la courbe (C) représente le pourcentage cumulé du DF-5HTP non transformé et de la DFS formée au cours du métabolisme du DF-5HTP.

On constate que le niveau de la DFS tombe à une valeur insignifiante au bout de 4 heures. Par contre, le taux du DF-5HTP reste considérable même après 24 heures.

D'autres études pharmacologiques ont été entreprises, en particulier pour apprécier la toxicité aigue chez la souris. Les divers produits étudiés sont administrés à doses croissantes en progression arithmétique à des lots de 5 souris femelles SWISS, EOPS, NMRI/Han pesant 23 à 24 g.

Les produits sont solubilisés dans l'eau distillée. Les DL 50 sont calculées par la méthode de BEHRENS (B.) et KARBER (C.). Le nombre d'animaux survivants dans les différents lots est vérifié 15 jours après l'administration des produits. La toxicité aigue (DL 50) exprimée en g/kg est supérieure à 5, avec la voie d'administration i.v. pour le DF-5HTP un produit représentatif selon l'invention.

On a également effectué des essais sur les tremblements qu'on constate après l'administration du 5-HTP en les comparant aux résultats obtenus avec le DF-5HTP selon l'invention. Les conditions expérimentales étaient les suivantes:

Chez des souris prétraitées par un inhibiteur de la MAO (Harmaline 25 mg/kg/ P.O.), l'injection de 5HTP (1 heure après l'IMAO) entraîne l'apparition de tremblements plus ou moins généralisés, accompagnés ou non d'hyperactivité motrice et qui sont dus à l'accumulation de sérotonine non dégradée au niveau des récepteurs post-synaptiques cérébraux. On peut établir pour chaque souris une cotation chiffrée suivant l'intensité des symptômes observés au cours du temps (cotation toutes les 5 min. entre la 5ème et la 45ème min. après l'injection de 5 HTP puis toutes les 15 min. jusqu'à la 75ème min.).

On additionne en une score total tous les chiffres obtenus aux différents temps sur des lots de 5 ou 10 souris.

Les actions du 5 HTP ont été comparées à celles du DF-5HTP.

Les résultats sont rassemblés dans le tableau 9 qui suit.

**TABLEAU 9**

| Produits | Doses mg/kg | Voie | Score total | Nombre d'animaux |
|---|---|---|---|---|
| 5 HTP | 75 | S.C. | 146 | 5 |
| DF-5HTP | 150* | S.C. | 0 | 5 |
| | 500 | S.C. | 0 | 5 |

* dose correspondant à 75 mg/kg de 5 HTP mole à mole

Dans ces conditions expérimentales le DF-5HTP se révèle inactif. Les activités indésirables du 5-HTP, par exemple tremblement, disparaissent en cas d'utilisation du DF-5HTP.

On a encore effectué des essais pour apprécier la quantité de sérotonine (5-HT) libérée dans le cerveau lors de l'administration des produits de l'invention par comparaison aux résultats obtenus avec le 5-HTP. Les quantités de sérotonine ont été estimées par une méthode spectrophotofluorométrique, telle que décrite par Solomon et al. Biochem. Pharmacology 14, 831 (1965). Les résultats de ces essais sont rassemblés dans le Tableau 10 ci-après.

**TABLEAU 10**

Quantités de 5-HT (dans tout le cerveau) après administration de 5-HTP et ses dérivés sucrés

| N° | Composé | Dose mg/kg i.p. | Quantités de 5-HT* 15 min. | 30 min.** | 60 min.** |
|---|---|---|---|---|---|
| 1. | Solution Saline | - | 60,0 | 107 | 100 |
| 2. | 5-HTP | 100 | 187,5 | 248,6 | 150,9 |
| 3. | DF-5HTP | 100 | 87,5 | - | - |
| 4. | DP-5HTP | 174 | 75,0 | 200,4 | 160,8 |
| 5. | D-Ribulo-5HTP | 160 | 92,5 | - | - |

* Chaque valeur représente la moyenne de quatre animaux d'expérience (souris)

** La concentration de 5-HT est exprimée par gramme de tissu frais (cerveau).

Les résultats du tableau 10 montrent notamment que des quantités équimoléculaires de 5-HTP (100 mg) et de DF-5HTP (175 mg) libèrent au bout d'une heure la même quantité de sérotonine dans le cerveau. Une telle observation est très importante d'autant qu'en même temps les effets secondaires indésirables du 5-HTP disparaissent lors de l'administration du DP-5HTP.

Il est également intéressant de noter qu'à une concentration de $10^{-4}$ Mol et à 25°C, la desoxyfructosérotonine (DF-5HT) inhibe à 90 % l'incorporation de la sérotonine (5-HT) dans les cellules des neuroblastomes in vitro, tandis que le DF-5HTP est sans effet à cette température sur ce phénomène. Ceci permet de supposer une restauration plus rapide des réserves de sérotonine de l'organisme en utilisamt le désoxyfructo-5-HTP.

Ces résultats sont mis en évidence dans le tableau 11 ci-après.

**TABLEAU 11**

**Inhibition de l'incorporation de la sérotonine dans les cellules nerveuses par la DF-5HT et le DF-5HTP**

| Inhibiteurs | % Inhibition à 25°C | % Inhibition à 0°C |
|---|---|---|
| DF-5HT | 90 | 95 |
| DF-5HTP | 0 | 50 |

En résumé, les résultats ci-dessus montrent que le DF-5HTP n'est pas un précurseur de la DFS et que, pour un effet anti-lépreux sensiblement équivalent, l'action du DF-5HTP se poursuit dans le temps largement au-delà de celle de la DFS. Cette propriété, ajoutée à la toxicité nettement moindre du DF-5HTP, prouve la supériorité du nouveau composé vis-à-vis de la DFS.

Ainsi, l'invention procure de nouveaux dérivés glucidiques du 5-hydroxytryptophane, en particulier le DF-5HTP, qui sont des agents antilèpreux de valeur.

**Revendications** pour les Etats Contractants: BE, CH, DE, GB, LU, NL, SE

1. Nouveaux dérivés glucidiques du 5-hydroxytryptophane (5-HTP) dans lequel l'un des atomes d'hydrogène du groupe amine primaire du 5-hydroxytryptophane est substitué par un groupe désoxy-i céto-2 sucre.

2. Dérivés glucidiques du 5-HTP selon la revendication 1 caractérisés en ce que le groupe sucré provient de l'un quelconquc des aldoses suivants; le glycérose, le thréose, l'érythrose, le ribose, l'arabinose, le xylose, le lyxose, le gulose, l'idose, le glucose, le maniose, le galactose, le talose, l'allose, l'altrose, le rahamnose, le fucose, le 6-désoxyglucose ou de disaccharides réducteurs, tels que le melibiose, le maltose, le cellobiose et le lactose.

3. Dérivés glucidiques du 5-HTP selon l'une des revendications 1 ou 2, caractérisés en ce que le groupe sucré appartient à la série D et/ou à la série L.

4. Dérivés glucidiques du 5-HTP selon l'une quelcongue des revendications 2 à 3, caractérisés en ce que le groupe sucré est choisi parmi les composés ci-après ou leurs isomères:
D-glucose (hexose),
D-ribose (pentose),
L-fucose (méthylpentose),
melibiose (disaccharide),
érythrose (tetrose).

5. Dérivés glucidiques du 5-HTP selon l'une quelconque des revendications 1 à 4 répondant à la formule:

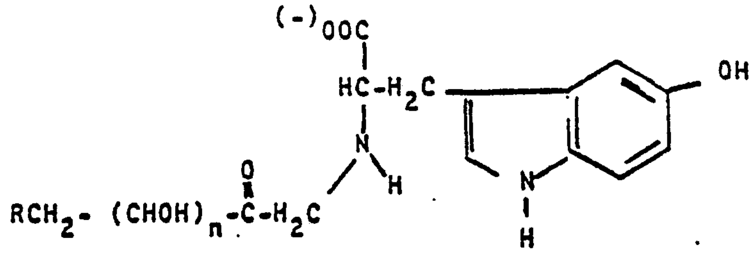

dans laquelle R représente un atome d'hydrogène ou un groupe hydroxyle et n est un nombre entier de 1 à 5.

6. Le N-1-désoxyfructo-5-hydroxytryptophane ou DF-5HTP de formule:

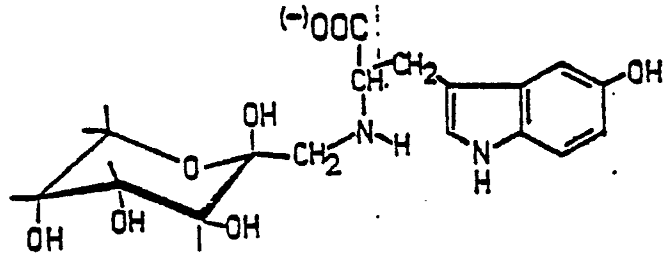

7. Dérivés glucidiques du 5-HTP selon l'une quelconque des revendications 1 à 6, sous forme libre (acide) ou sous forme de leurs sels, en particulier les sels pharmaceutiquement acceptables, tels que les sels alcalins et alcalino-terreux, les sels d'ammonium, ou avec des amines primaires, secondaires ou tertiaires.

8. Procédé pour l'obtention des dérivés glucidiques du 5-HTP selon l'une quelconcue des revendications 1 à 7 par une réaction de type Amadori, ledit procédé étant caractérisé en ce qu'on met en présence au sein d'un solvant le 5-hydroxytryptophane et le sucre correspondant au dérivé sucré à préparer.

9. Procédé selon la revendication 8, caractérisé en ce que le solvant est un alcool, tel qu'un alcool inférieur, par exemple le méthanol, l'éthanol, le propanol ou l'isopropanol ou le dioxane, le diméthylformamide ou l'acétonitrile ou des mélanges compatibles de tels solvants, ou tout autre solvant usuel pour la synthèse des dérivés Amadori.

10. Procédé selon l'une des revendications 8 ou 9, caractérisé en ce qu'on utilise un excès de sucre par rapport au 5-HTP, par exemple environ 1,5 mole de sucre par mole de 5-HTP.

11. Procédé selon l'une quelconque des revendications 8 à 10, caractérisé en ce qu'on travaille dans une gamme de température allant de la température ambiante jusqu'à la température de reflux du solvant, cette dernière étant préférée.

12. Application des dérivés glucidiques du 5-HTP selon l'une quelconque des revendications 1 à 7 et des composés obtenus par le procédé selon l'une quelconque des revendications 8 à 11 à la fabrication d'un médicament.

13. Médicament contre la lèpre contenant à titre d'agent actif, au moins un dérivé glucidique du 5-HTP selon l'une quelconque des revendications 1 à 7 ou un des composés obtenus par le procédé selon l'une quelconque des revendications 8 à 11.

14. Composition pharmaceutique contenant une quantité efficace d'au moins un composé de l'invention ou d'un de ses sels physiologiquement acceptables en association avec un véhicule convenant pour

l'administration considérée.

15. Composition pharmaceutique selon la revendication 13 pour le traitement de la lèpre.

16. Compositions pharmaceutiques selon l'une des revendications 13 ou 14 convenant à l'administration orale et présentées, notamment, sous forme de gélules ou de comprimés, ou bien à l'administration locale et présentées, notamment, sous forme de lotions ou pommades ou bien encore à l'injection sous-cutanée et intraveineuse.

17. Composition pharmaceutique selon l'une quelconque des revendications 13 à 15, pour le traitement de la lèpre, procurant une quantité efficace d'agent actif allant jusqu'à 20 mg par kg de poids et par jour, des doses de 2 à 10 mg/kg/jour étant préférées.

**Revendications** pour l'Etat Contractant: AT

1. Procédé pour l'obtention de nouvenux dérivés glucidiques du 5-hydroxytryptophane (5-HTP) dans lequel l'un des atomes d'hydrogène du groupe amine primaire du 5-hydroxytryptophane est substitué par un groupe désoxy-1 céto-2 sucre, par une réaction de type Amadori, ledit procédé étant caractérisé en ce qu'on met en présence, au sein d'un solvant, le 5-hydroxytryptophane et le sucre correspondant au dérivé sucré à préparer.

2. Procédé selon la revendication 1, caractérisé en ce que le groupe sucré provient de l'un quelconque des aldoses suivants: le glycérose, le thréose, l'érythrose, le ribose, l'arabinose, le xylose, le lyxose, le gulose, l'idose, le glucose, le mannose, le galactose, le talose, l'allose, l'altrose, le rhamnose, le fucose, le 6-désoxyglucose ou de disaccharides réducteurs, tels que le melibiose, le maltose, le cellobiose et le lactose.

3. Procédé selon l'une des revendications 1 ou caractérisé en ce que le groupe sucré appartient à la série D et/ou à la serie L.

4. Procédé selon l'une quelconque des revendications 2 et 3, caractérisé en ce que le groupe sucré est choisi parmi les composés ci-après ou leurs isomères:

D-glucose (hexose),
D-ribose (pentose),
L-fucose (méthylpentose),
melibiose (disaccharide),
érythrose (tetrose).

5. Procédé selon l'une quelconque des revendications 1 à 4, caractérisé en ce que le dérivé glucidique du 5-hydroxytryptophane obtenu repond à la formule:

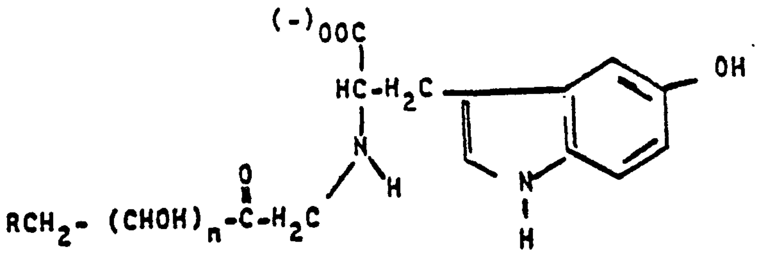

dans laquelle R représente un atome d'hydrogène ou un groupe hydroxyle et n est un nombre entier de 1 à 5.

6. Procédé selon la revendication 1, caractérisé en ce que le dérivé glucidique du 5-hydroxytryptophane obtenu est le N-1-désoxyfructo-5-hydroxytryptophane ou DP-5HTP de formule:

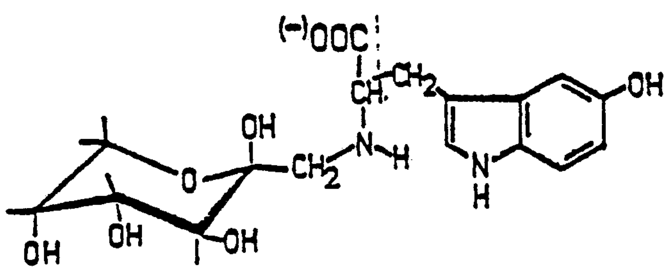

7. Procédé selon l'une des revendications 1 à 6, caractérisé en ce que l'on obtient les dérivés sous forme libre (acide) ou sous forme de leurs sels, en particulier les sels pharmaceutiquement acceptables, tels que les sels alcalins et alcalino-terreux, les sels d'ammonium, ou avec des amines primaires, secondaires ou tertiaires.

8. Procédé selon l'une des revendications 1 à 7, caractérisé en ce que le solvant est un alcool, tel qu'un alcool inférieur, par exemple le méthanol, l'éthanol, le propanol ou l'isopropanol ou le dioxane, le diméthylformamide ou l'acétonitrile ou des mélanges compatibles de tels solvants, ou tout autre solvant usuel pour la synthèse des dérivés Amadori.

9. Procédé selon l'une des revendications 1 à 8, caractérisé en ce qu'on utilise un excès de sucre par rapport

au 5-HTP, par exemple environ 1,5 mole de suore par mole de 5-HTP.

10. Procédé selon l'une quelconque des revendications 1 à 9, caractérisé en ce qu'on travaille dans une gamme de température allant de la température ambiante jusqu'à la température de reflux du solvant, cette dernière étant préférée.

11. Procédé pour la préparation d'une composition pharmaceutique qui consiste à formuler au moins un dérivé glucidique de 5-HTP obtenu par le procédé tel que défini à l'une quelconque des revendications 1 à 10, avec un véhicule pharmaceutiquement acceptable.

12. Procédé pour la préparation d'une composition pharmaceutique destinée au traitement de la lèpre, caractérisé en ce qu'il consiste à formuler au moins un dérivé glucidique de 5-HTP obtenu par le procédé tel que défini à l'une quelconque des revendications 1 à 10, avec un véhicule pharmaceutiquement acceptable.

13. Procédé selon l'une des revendications 11 et 12, caractérisé en ce qu'on prépare une composition administrable par voie orale et présentée, notamment, sous forme de gélules ou de comprimés, ou bien par voie locale et présentée, notamment, sous forme de lotions ou pommades ou bien encore par injection sous-cutanée et intra-veineuse.

14. Procédé selon l'une des revendications 12 et 13, caractérisé en ce qu'on prépare une composition procurant une quantité efficace d'agent actif allant jusqu'à 20 mg par kg de poids et par jour, des doses de 2 à 10 mg/kg/jour étant préférées.

**Patentansprüche** für die Vertragsstaaten: BE, CH, DE, GB, LU, NL, SE

1. Neue Glucidderivate des 5-Hydroxytryptophans (5-HTP) in denen eines der Wasserstoffatome der primären Amingruppe des 5-Hydroxytryptophans durch eine Desoxy-1 keto-2 Zuckergruppe substituiert ist.

2. Glucidderivate des 5-HTP gemäß Anspruch 1, dadurch gekennzeichnet, daß sich die zuckerähnliche Gruppe von irgendeiner der folgenden Aldosen ableitet: der Glycerose, der Threose, der Erythrose, der Ribose, der Arabinose, der Xylose, der Lyxose, der Gulose, der Idose, der Glucose, der Mannose, der Galactose, der Talose, der Allose, der Altrose, der Rahamnose, der Fucose, der 6-Desoxyglucose oder reduzierenden Disacchariden wie der Melibiose, der Maltose, der Cellobiose und der Lactose.

3. Glucidderivate des 5-HTP gemäß einem der Ansprüche 1 oder 2, dadurch gekennzeichnet, daß die zuckerähnliche Gruppe der D-Reihe und/oder der L-Reihe angehört.

4. Glucidderivate des 5-HTP gemäß irgendeinem der Ansprüche 2 oder 3, dadurch gekennzeichnet, daß die zuckerähnliche Gruppe aus folgenden Verbindungen oder ihren Isomeren ausgewählt ist:
D-Glucose (Hexose),
D-Ribose (Pentose),
L-Fucose (Methylpentose)
Melibiose (Disaccharid),
Erythrose (Tetrose).

5. Glucidderivate des 5-HTP gemäß irgendeinem der Ansprüche 1 bis 4, die folgende Formel aufweisen:

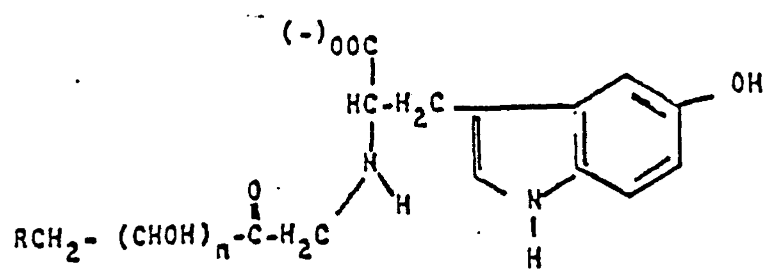

in der R für ein Wasserstoffatom oder eine Hydroxylgruppe steht und n eine ganze Zahl von 1 bis 5 ist.

6. Das N-1-Desoxyfructo-5-hydroxytryptophan oder DF-5-HTP der Formel:

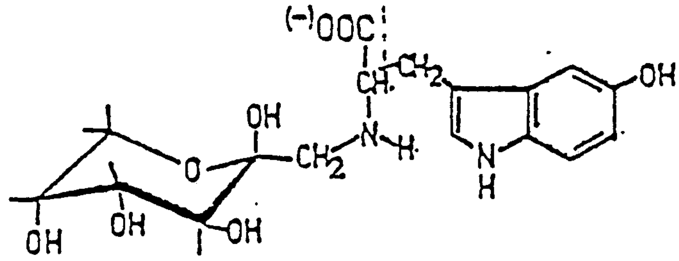

7. Glucidderivate des 5-HTP gemäß irgendeinem der Ansprüche 1 bis 6, in freier Form (Säure) oder in Form ihrer Salze, insbesondere ihrer pharmazeutisch verträglichen Salze, wie ihrer Alkali- und Erdalkalisalze, ihrer Ammoniumsalze, oder mit primären, sekundären oder tertiären Aminen.

8. Verfahren zur Herstellung eines Glucidderivates des 5-HTP gemäß irgendeinem der Ansprüche 1 bis 7 mittels einer Reaktion des Typs Amadori, das dadurch gekennzeichnet ist, daß man das 5-Hydroxytryptophan und einen Zucker, der dem zuckerähnlichen Derivat entspricht, in einem Lösungsmittel umsetzt.

9. Verfahren gemäß Anspruch 8, dadurch gekennzeichnet, daß das Lösungsmittel ein Alkohol wie ein niederer Alkohol, beispielsweise das Methanol, das Ethanol, das Propanol oder das Isopropanol oder das Dioxan, das Dimethylformamid oder das Acetonitril oder kompatible Mischungen dieser Lösungsmittel, oder ganz andersartige Lösungsmittel wie für die Synthese von Amadoriderivaten üblich, sind.

10. Verfahren gemäß einem der Ansprüche 8 oder 9, dadurch gekennzeichnet, daß man einen Überschuß an Zucker im Vergleich zum 5-HTP verwendet, beispielsweise ca. 1,5 Mol Zucker pro Mol 5-HTP.

11. Verfahren gemäß irgendeinem der Ansprüche 8 bis 10, dadurch gekennzeichnet, daß man innerhalb eines Temperaturbereichs arbeitet, welcher von Raumtemperatur bis zur Rückflußtemperatur des Lösungsmittels reicht, wobei diese letztere bevorzugt ist.

12. Verwendung von Glucidderivaten des 5-HTP gemäß irgendeinem der Ansprüche 1 bis 7 sowie von Verbindungen, die gemäß dem Verfahren gemäß irgendeinem der Ansprüche 8 bis 11 erhalten worden sind, zur Herstellung eines Arzneimittels.

13. Arzneimittel gegen Lepra, enthaltend als Wirkstoff wenigstens ein Glucidderivat des 5-HTP gemäß irgendeinem der Ansprüche 1 bis 7 oder einer Verbindung, welche erhalten worden ist gemäß des Verfahrens gemäß irgendeinem der Ansprüche 8 bis 11.

14. Pharmazeutische Zusammensetzung, enthaltend eine wirksame Menge wenigstens einer der erfindungsgemäßen Verbindungen oder deren physiologisch annehmbaren Salzen in Verbindung mit einem für die beabsichtigte Verabreichung verträglichen Vehikel.

15. Pharmazeutische Zusammensetzung gemäß Anspruch 13 zur Behandlung von Lepra.

16. Pharmazeutische Zusammensetzung gemäß einem der Ansprüche 13 oder 14 geeignet zur oralen Verabreichung, insbesondere in Form von Gelatinekapseln oder Tabletten oder auch zur lokalen Verabreichung, insbesondere in Form von Lotionen oder Salben oder weiterhin auch zur subkutanen und intravenösen Injektion.

17. Pharmazeutische Zusammensetzung gemäß irgendeinem der Ansprüche 13 bis 15 zur Behandlung von Lepra, liefernd eine wirksame Menge eines Wirkstoffs von bis zu 20 mg pro kg Körpergewicht und Tag, wobei eine Dosis von 2 bis 10 mg pro kg und Tag bevorzugt ist.

**Patentansprüche** für den Vertragsstaat: AT

1. Verfahren zur Herstellung von neuen Glucidderivaten des 5-Hydroxytryptophans (5-HTP), in denen eines der Wasserstoffatome der primären Amingruppe des 5-Hydroxytryptophans durch eine Desoxy-1 keto-2 Zuckergruppe substituiert worden ist, mittels einer Reaktion des Typs Amadori, wobei besagter Prozeß dadurch gekennzeichnet ist, daß man das 5-Hydroxy-Tryptophan und den Zucker, der dem zuckerähnlichen Derivate entspricht, in einem Lösungsmittel umsetzt.

2. Verfahren gemäß Anspruch 1, dadurch gekennzeichnet, daß die zuckerähnliche Gruppe aus irgendeinem der folgenden Aldosen ausgewählt ist: der Glycerose, der Threose, der Erythrose, der Ribose, der Arabinose, der Xylose, der Lyxose, der Gulose, der Idose, der Glucose, der Mannose, der Galactose, der Talose, der Allose, der Altrose, der Rahamnose, der Fucose, der 6-Desoxyglucose oder reduzierenden Disacchariden wie der Melibiose, der Maltose, der Cellobiose und der Lactose.

3. Verfahren gemäß einem der Ansprüche 1 oder 2, dadurch gekennzeichnet, daß die zuckerähnliche Gruppe der D-Reihe und/oder der L-Reihe angehört.

4. Verfahren gemäß irgendeinem der Ansprüche 2 und 3, dadurch gekennzeichnet, daß die zuckerähnliche Gruppe aus folgenden Verbindungen oder ihren Isomeren ausgewählt ist:

D-Glucose (Hexose),

D-Ribose (Pentose),

L-Fucose (Methylpentose)
Melibiose (Disaccharid),
Erythrose (Tetrose).

5. Verfahren gemäß irgendeinem der Ansprüche 1 bis 4, dadurch gekennzeichnet, daß das erhaltene Glucidderivat des 5-Hydroxytryptophans folgende Formel aufweist:

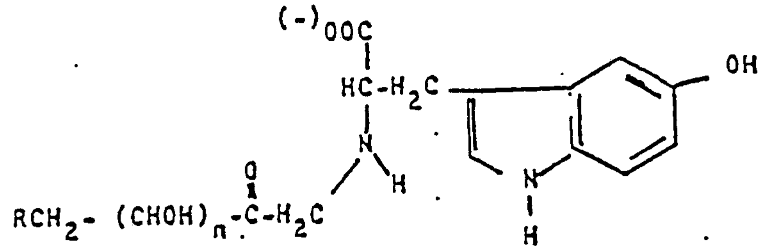

in der R für ein Wasserstoffatom oder eine Hydroxylgruppe steht und n eine ganze Zahl von 1 bis 5 ist.

6. Verfahren gemäß Anspruch 1, dadurch gekennzeichnet, daß das erhaltene Glucidderivat des 5-Hydroxytryptophans das N-1-Desoxyfructo-5-hydroxytryptophan oder DF-5-HTP der Formel

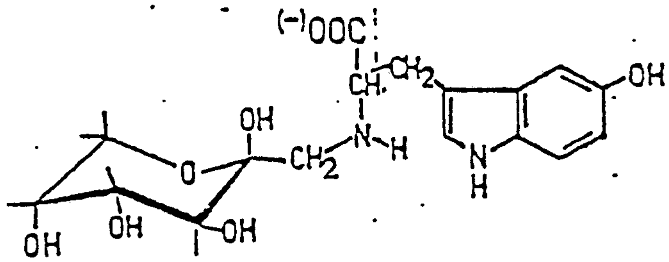

ist.

7. Verfahren gemäß einem der Ansprüche 1 bis 6, dadurch gekennzeichnet, daß man die Derivate in freier Form (Säure) oder in Form ihrer Salze, insbesondere ihrer pharmazeutisch verträglichen Salze, wie der Alkali- und Erdalkalisalze, der Ammoniumsalze, oder mit primären, sekundären oder tertiären Aminen, erhält.

8. Verfahren gemäß irgendeinem der Ansprüche 1 bis 7, dadurch gekennzeichnet, daß das Lösungsmittel ein Alkohol wie ein niederer Alkohol, beispielsweise das Methanol, das Ethanol, das Propanol oder das Isopropanol oder das Dioxan, das Dimethylformamid oder das Acetonitril oder kompatible Mischungen dieser Lösungsmittel, oder ganz andersartige Lösungsmittel wie für die Synthese von Amadoriderivaten üblich, sind.

9. Verfahren gemäß einem der Ansprüche 1 bis 8, dadurch gekennzeichnet, daß man einen Überschuß an Zucker im Vergleich zum 5-HTP verwendet, beispielsweise ca. 1,5 Mol Zucker pro Mol 5-HTP.

10. Verfahren gemäß irgendeinem der Ansprüche 1 bis 9, dadurch gekennzeichnet daß man innerhalb eines Temperaturbereichs arbeitet, welcher von Raumtemperatur bis zur Rückflußtemperatur des Lösungsmittels reicht, wobei diese letztere bevorzugt ist.

11. Verfahren zur Herstellung einer pharmazeutischen Zusammensetzung, die darin besteht, daß man wenigstens ein Glucidderivat des 5-HTP, erhalten gemäß eines Verfahrens wie es in einem der Ansprüche 1 bis 10 definiert worden ist, zusammen mit einem pharmazeutisch verträglichen Vehikel formuliert.

12. Verfahren zur Herstellung einer pharmazeutischen Zusammensetzung für die Behandlung von Lepra, dadurch gekennzeichnet, daß man wenigstens ein Glucidderivat des 5-HTP, erhalten gemäß eines Verfahrens wie es in einem der Ansprüche 1 bis 10 definiert worden ist, zusammen mit einem pharmazeutisch verträglichen Vehikel formuliert.

13. Verfahren gemäß einem der Ansprüche 11 und 12, dadurch gekennzeichnet, daß man eine oral verabreichbare Zusammensetzung, insbesondere in Form von Gelatinekapseln oder Tabletten oder eine lokal verabreichbare Zusammensetzung, insbesondere in Form von Lotionen oder Salben oder eine durch subkutane oder intravenöse Injektion verabreichbare Zusammensetzung herstellt.

14. Verfahren gemäß einem der Ansprüche 12 und 13, dadurch gekennzeichnet, daß man eine Zusammensetzung zubereitet, liefernd eine wirksame Menge eines Wirkstoffs, die bis zu 20 mg pro kg Körpergewicht und Tag beträgt, wobei eine Dosis von 2 bis 10 mg pro kg pro Tag bevorzugt ist.

**Claims** for the Contracting States: BE, CH, DE, GB, LU, NL, SE

1. New carbohydrate derivatives of 5-hydroxytryptophan (5-HTP) in which one of the hydrogen atoms of the primary amine group of 5-hydroxytryptophan is substituted with a 1-deoxy-2-keto sugar group.

2. Carbohydrate derivatives of 5-HTP according to Claim 1, characterized in that the sugar group originates from any one of the following aldoses: glycerose, threose, erythrose, ribose, arabinose, xylose, lyxose, gulose, idose, glucose, mannose, galactose, talose, allose, altrose, rhamnose, fucose and 6-deoxyglucose; or from reducing disaccharides such as melibiose, maltose, cellobiose and lactose.

3. Carbohydrate derivatives of 5-HTP according to one of Claims 1 and 2, characterized in that the sugar group belongs to the D serie and/or to the L serie.

4. Carbohydrate derivatives of 5-HTP according to either of Claims 2 and 3, characterized in that the sugar group is chosen from the compounds below or their isomers:

D-glucose (hexose),
D-ribose (pentose),
L-fucose (methylpentose),
melibiose (disaccharide),
erythrose (tetrose),

5. Carbohydrate derivatives of 5-HTP according to any one of Claims 1 to 4, corresponding to the formula:

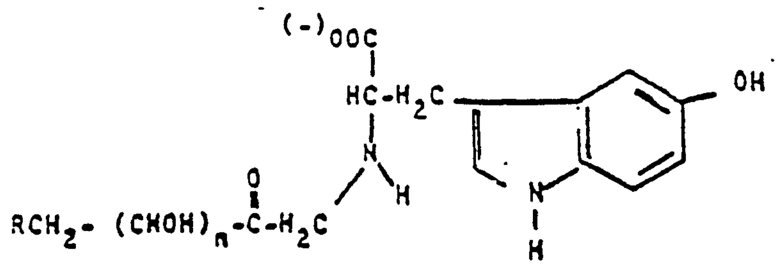

in which R denotes a hydrogen atom or a hydroxyl group and n is an integer from 1 to 5.

6. N-(1-deoxyfructo)-5-hydroxytryptophan or DF-5HTP of formula

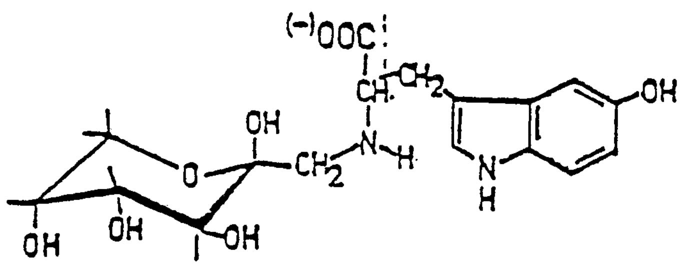

7. Carbohydrate derivatives of 5-HTP according to any one of Claims 1 to 6, in free (acid) form or in the form of their salts, especially the pharmaceutically acceptable salts such as the alkali metal and alkaline earth metal salts, ammonium salts or salts with primary, secondary or tertiary amines.

8. Process for obtaining the carbohydrate derivatives of 5-HTP according to any one of Claims 1 to 7 by an Amadori type reaction, the said process being characterized in that 5-hydroxytryptophan and the sugar corresponding to the sugar derivative to be prepared are brought into contact in a solvent.

9. Process according to Claim 8, characterized in that the solvent is an alcohol such as a lower alcohol, for example methanol, ethanol, propanol or isopropanol, or dioxane, dimethylformamide or acetonitrile, or compatible mixtures of such solvents, or any other solvent usual for the synthesis of Amadori derivatives.

10. Process according to one of Claims 8 and 9, characterized in that an excess of sugar relative to 5-HTP, for example approximately 1.5 mole of sugar per mole of 5-HTP, is used.

11. Process according to any one of Claims 8 to 10, characterized in that the reaction is performed within a temperature range extending from room temperature to the refluxing temperature of the solvent, the latter being preferred.

12. Application of the carbohydrate derivatives of 5-HTP according to any one of Claims 1 to 7, and of the compounds obtained by the process according to any one of Claims 8 to 11, to the manufacture of a medicinal product.

13. Medicinal product against leprosy containing, as active agent, at least one carbohydrate derivative of 5-HTP according to any one of Claims 1 to 7 or one of the compounds obtained by the process according to any one of Claims 8 to 11.

14. Pharmaceutical composition containing an effective amount of at least one compound of the invention, or

of one of its physiologically acceptable salts, in combination with a vehicle suitable for the administration in question.

15. Pharmaceutical composition according to Claim 13 for the treatment of leprosy.

16. Pharmaceutical compositions according to one of Claims 13 and 14, which are suitable for oral administration and presented, in particular, in the form of capsules or of tablets, or alternatively for local administration and presented, in particular, in the form of lotions or ointments, or alternatively for subcutaneous and intravenous injection.

17. Pharmaceutical composition according to any one of Claims 13 to 15, for the treatment of leprosy, providing an effective amount of active agent ranging up to 20 mg per kg of weight and per day, doses of 2 to 10 mg/kg/day being preferred.

**Claims** for the Contracting State: AT

1. Process for obtaining new carbohydrate derivatives of 5-hydroxytryptophan (5-HTP) in which one of the hydrogen atoms of the primary amine group of 5-hydroxytryptophan is substituted with a 1-deoxy-2-keto sugar group, by an Amadori type reaction, characterized in that 5-hydroxytryptophan and the sugar corresponding to the sugar derivative to be prepared are brought into contact in a solvent.

2. Process according to Claim 1, characterized in that the sugar group originates from any one of the following aldoses: glycerose, threose, erythrose, ribose, arabinose, xylose, lyxose, gulose, idose, glucose, mannose, galactose, talose, allose, altrose, rhamnose, fucose and 6-deoxyglucose; or from reducing disaccharides such as melibiose, maltose, cellobiose and lactose.

3. Process according to one of Claims 1 and 2, characterized in that the sugar group belongs to the D serie and/or to the L serie.

4. Process according to any one of Claims 2 and 3, characterized in that the sugar group is chosen from the compounds below or their isomers:

D-glucose (hexose),
D-ribose (pentose),
L-fucose (methylpentose),
melibiose (disaccharide),
erythrose (tetrose).

5. Process according to any of Claims 1 to 4, characterized in that the carbohydrate derivative of 5-hydroxytryptophan obtained corresponds to the formula:

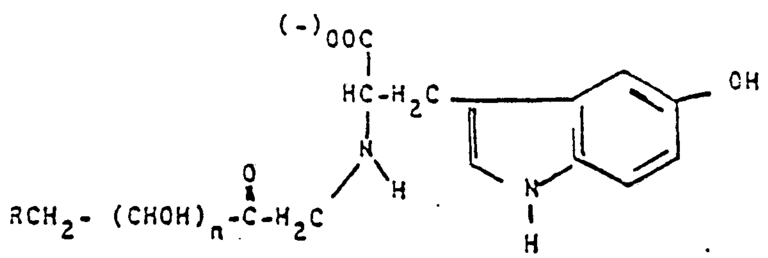

in which R denotes a hydrogen atom or a hydroxyl group and n is an integer from 1 to 5.

6. Process according to Claim 1, characterized in that the carbohydrate derivative of 5-hydroxytryptophan obtained is N-(1-deoxyfructo)-5-hydroxytryptophan or DF-5HTP of formula:

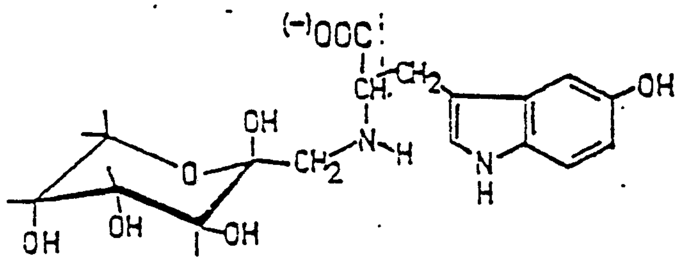

7. Process according to one of Claims 1 to 6, characterized in that the derivatives are obtained in free (acid) form or in the form of their salts, especially the pharmaceutically acceptable salts such as the alkali metal and alkaline earth metal salts, ammonium salts or salts with primary, secondary or tertiary amines.

8. Process according to one of Claims 1 to 7, characterized in that the solvent is an alcohol such as a lower

alcohol, for example methanol, ethanol, propanol or isopropanol, or dioxane, dimethylformamide or acetonitrile, or compatible mixtures of such solvents, or any other solvent usual for the synthesis of Amadori derivatives.

9. Process according to one of Claims 1 to 8, characterized in that an excess of sugar relative to 5-HTP, for example approximately 1.5 mole of sugar per mole of 5-HTP, is used.

10. Process according to any one of Claims 1 to 9, characterized in that the reaction is performed within a temperature range extending from room temperature to the refluxing temperature of the solvent, the latter being preferred.

11. Process for preparing a pharmaceutical composition, which consists in formulating at least one carbohydrate derivative of 5-HTP obtained by the process as defined in any one of Claims 1 to 10, with a pharmaceutically acceptable vehicle.

12. Process for preparing a pharmaceutical composition intended for the treatment of leprosy, characterized in that it consists in formulating at least one carbohydrate derivative of 5-HTP obtained by the process as defined in any one of Claims 1 to 10, with a pharmaceutically acceptable vehicle.

13. Process according to one of Claims 11 and 12, characterized in that a composition is prepared which is administrable orally and presented, in particular, in the form of capsules or of tablets, or alternatively locally and presented, in particular, in the form of lotions or ointments, or alternatively by subcutaneous and intravenous injection.

14. Process according to one of Claims 12 and 13, characterized in that a composition is prepared that provides an effective amount of active agent ranging up to 20 mg per kg of weight and per day, doses of 2 to 10 mg/kg/day being preferred.

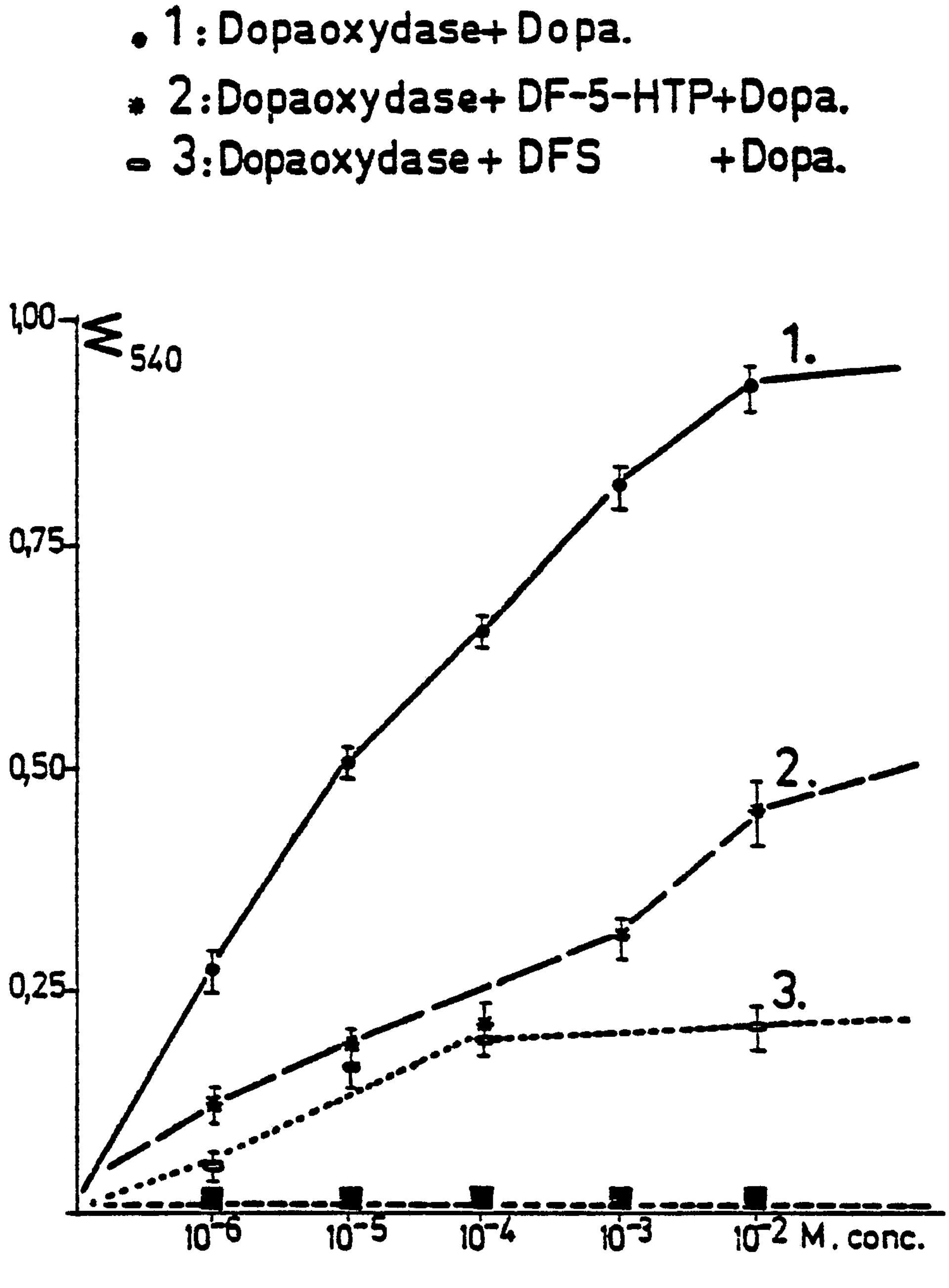
• 1 : Dopaoxydase+ Dopa.
* 2 : Dopaoxydase+ DF-5-HTP+Dopa.
▭ 3 : Dopaoxydase + DFS +Dopa.
1,00
0,75
0,50
0,25
ε 540
1.
2.
3.
10⁻⁶
10⁻⁵
10⁻⁴
10⁻³
10⁻² M. conc.

FIG.1

FIG.2